# EUROPEAN PATENT APPLICATION

(11) **EP 2 982 374 A1**
(43) Date of publication of application: **10.02.2016**
(21) Application number: 13881188.0
(22) Date of filing: 01.04.2013
(51) Int. Cl.: A61K 35/18

(54) **RED BLOOD CELL STORAGE SOLUTION, STORAGE SOLUTION ACCOMMODATING CONTAINER, MANUFACTURING METHOD FOR RED BLOOD CELL STORAGE SOLUTION, AND RED BLOOD CELL BAG SYSTEM**

(71) Applicant: Terumo Kabushiki Kaisha, Tokyo 151-0072 (JP)
(72) Inventor: TAKEDA Norihiko, Fujinomiya-shi Shizuoka 418-0004 (JP)
(74) Representative: Casalonga
(86) International application number: PCT/JP2013/059900
(87) International publication number: WO 2014/162435

(57) **Abstract**

A red blood cell preservative includes: a first aqueous solution containing mannitol, adenine, sodium citrate, sodium dihydrogen phosphate, and dextrose; and a second aqueous solution containing disodium phosphate, characterized in that the first aqueous solution and the second aqueous solution are mixed together after autoclave sterilization.

## Description

### Technical Field

The present invention relates to a red blood cell preservative to be used in order to store concentrated red blood cells over an extended period of time; a preservative container to contain the red blood cell preservative; a manufacturing method for red blood cell preservative using the preservative container; and a blood bag system including the preservative container.

### Background Art

Red blood cell preservatives have been conventionally used in order to store concentrated red blood cells over an extended period of time, an example of which being described in Patent Literature 1. Such red blood cell preservatives are aqueous solutions containing glucose, sodium citrate, disodium phosphate, sodium dihydrogen phosphate, adenine, and/or mannitol with a buffer that is biologically compatible with red blood cells. Because such red blood cell preservatives contain glucose (dextrose), the heat from autoclave sterilization in the course of manufacture causes denaturation and caramelization of the dextrose, thereby disadvantageously browning the red blood cell preservatives.

In order to address this problem, Patent Literature 2 describes that a red blood cell storage solution (red blood cell preservative) is formed of two different aqueous solutions, which are contained in different containers, respectively, prior to autoclave sterilization, and are mixed together after autoclave sterilization. Specifically, a first container contains a first aqueous solution containing dextrose, while a second container contains a second aqueous solution containing the other preservative ingredients, i.e., adenine, mannitol, sodium citrate, sodium dihydrogen phosphate, and disodium phosphate.

### Citation List

### Patent Literatures

Patent Literature 1: JP 3069613 B2
Patent Literature 2: JP 3185108 B2

### Summary of Invention

### Technical Problem

In Patent Literature 2, the pH of the first aqueous solution containing dextrose will be in an acidic range because it contains dextrose only. As a result, dextrose is less prone to denaturation, whereby browning of the red blood cell preservative is reduced. Patent Literature 2, however, has a problem: because the second aqueous solution contained in the second container contains disodium phosphate, the pH of the second aqueous solution is increased, which makes other preservative ingredients than disodium phosphate easily adsorb to the container wall, whereby the effectiveness of the second solution and the red blood cell preservative is decreased. Furthermore, another problem is that, the increase in the pH of the second aqueous solution decreases the solubility of the organic ingredients such as adenine, thereby complicating the preparation process of the second aqueous solution.

The present invention has been made in order to solve the above problems with an object of providing a red blood cell preservative, in which denaturation (browning) of dextrose by the heat from sterilization is sufficiently reduced, and the effectiveness of the red blood cell preservative is not decreased and the preparation thereof is easily done. Furthermore, the invention has another object of providing a preservative container, a manufacturing method for red blood cell preservative, and a blood bag system, in which browning of the red blood cell preservative by the heat from sterilization is sufficiently reduced, and the effectiveness of the red blood cell preservative is not decreased and preparation thereof is easily done.

### Solution to Problem

In order to solve the above problems, a red blood cell preservative of the invention includes: a first aqueous solution containing mannitol, adenine, sodium citrate, sodium dihydrogen phosphate, and dextrose; and a second aqueous solution containing disodium phosphate, characterized in that the first aqueous solution and the second aqueous solution are mixed together after autoclave sterilization.

In the red blood cell preservative of the invention, the first aqueous solution containing dextrose will have a pH within an acidic range because it does not contain disodium phosphate that would keep the pH within an alkaline range. Consequently, dextrose will not be denatured by the heat from autoclave sterilization. Furthermore, because the pH of the first aqueous solution is within an acidic range, the adsorption of the preservative ingredients in the first aqueous solution to the container wall is reduced, and thus the solubility of the organic ingredients such as adenine in the first aqueous solution will not decrease. Furthermore, the mixing of the first aqueous solution and the second aqueous solution happening after autoclave sterilization makes the red blood cell preservative have improved storability of red blood cells.

A preservative container of the invention contains the red blood cell preservative and includes a bag-type container formed of a flexible material, characterized in that: the bag-type container includes a weak seal for partitioning its internal space into a first preservative chamber and a second preservative chamber; the first preservative chamber contains the first aqueous solution and the second preservative chamber contains the second aqueous solution; and, after autoclave sterilization, the first preservative chamber or the second preservative chamber is depressed to peel the weak seal such that the first aqueous solution and the second aqueous solution are mixed together.

In the preservative container of the invention, the first preservative chamber containing the first aqueous solution containing dextrose is separated from the second preservative chamber containing the second aqueous solution containing disodium phosphate by the weak seal; therefore, the pH of the first aqueous solution is kept within an acidic range, whereby denaturation of dextrose by the heat from autoclave sterilization is preventable. Furthermore, adsorption of the preservative ingredients in the first aqueous solution to the inner wall of the first preservative chamber is reduced, and thus the solubility of the organic ingredients such as adenine in the first aqueous solution will not decrease. Furthermore, the mixing of the first aqueous solution and the second aqueous solution by peeling of the weak seal happening after autoclave sterilization makes the red blood cell preservative have improved storability of red blood cells.

A preservative container of the invention contains the red blood cell preservative and includes: a first bag-type container that is formed of a flexible material and contains the first aqueous solution; and a second bag-type container that is formed of a flexible material and contains the second aqueous solution; and a connecting tube that is formed of a flexible material and interconnects the first bag-type container and the second bag-type container, characterized in that, after autoclave sterilization, the first aqueous solution and the second aqueous solution are mixed together via the connecting tube.

In the preservative container of the invention, the first aqueous solution containing dextrose is contained in the first bag-type container, which is a separate container from the second bag-type container containing the second aqueous solution containing disodium phosphate; therefore, the pH of the first aqueous solution is kept within an acidic range, whereby denaturation of dextrose by the heat from autoclave sterilization is preventable. Furthermore, adsorption of the preservative ingredients in the first aqueous solution to the inner wall of the first bag-type container is reduced, and thus the solubility of the organic ingredients such as adenine in the first aqueous solution will not decrease. Furthermore, the mixing of the first aqueous solution and the second aqueous solution via the connecting tube happening after autoclave sterilization makes the red blood cell preservative have improved storability of red blood cells.

A manufacturing method for red blood cell preservative of the invention is for manufacturing the above-mentioned red blood cell preservative using the above-mentioned preservative container that is partitioned into the first preservative chamber and the second preservative chamber, characterized by including: a first step of containing the first aqueous solution in the first preservative chamber and the second aqueous solution in the second preservative chamber, respectively; following the first step, a second step of autoclaving the preservative container containing the first aqueous solution and the second aqueous solution; and following the second step, a third step of mixing the first aqueous solution and the second aqueous solution by peeling the seal by depressing the first preservative chamber or the second preservative chamber.

In the first step of the manufacturing method for red blood cell preservative of the invention, the first aqueous solution containing dextrose is contained in the first preservative chamber, which is separated from the second preservative chamber containing the second aqueous solution containing disodium phosphate by the weak seal; therefore, the pH of the first aqueous solution is kept within an acidic range, whereby denaturation of dextrose by the heat from autoclave sterilization in the second step is preventable. Furthermore, adsorption of the preservative ingredients in the first aqueous solution to the inner wall of the first preservative chamber is reduced, and thus the solubility of the organic ingredients such as adenine in the first aqueous solution will not decrease. Furthermore, in the third step, the mixing of the first aqueous solution and the second aqueous solution by peeling of the weak seal happening after autoclave sterilization makes the red blood cell preservative have improved storability of red blood cells.

A manufacturing method for red blood cell preservative of the invention is for manufacturing the above-mentioned red blood cell preservative using the above-mentioned preservative container including the first bag-type container and the second bag-type container, characterized by including: a first step of containing the first aqueous solution in the first bag-type container and the second aqueous solution in the second bag-type container, respectively; following the first step, a second step of autoclaving the preservative container containing the first aqueous solution and the second aqueous solution; and following the second step, a third step of mixing the first aqueous solution and the second aqueous solution via the connecting tube. Following the third step, the manufacturing method for red blood cell preservative may further include a fourth step of separating the second bag-type container from the first bag-type container by cutting the connecting tube.

In the first step of the manufacturing method for red blood cell preservative of the invention, the first aqueous solution containing dextrose is contained in the first bag-type container, which is a separate container from the second bag-type container containing the second aqueous solution containing disodium phosphate; therefore, the pH of the first aqueous solution is kept within an acidic range, whereby denaturation of dextrose by the heat from autoclave sterilization in the second step is preventable. Furthermore, adsorption of the preservative ingredients in the first aqueous solution to the inner wall of the first bag-type container is reduced, and thus the solubility of the organic ingredients such as adenine in the first aqueous solution will not decrease. Furthermore, in the third step, the mixing of the first aqueous solution and the second aqueous solution via the connecting tube happening after autoclave sterilization makes the red blood cell preservative have improved storability of red blood cells.

A blood bag system of the invention includes the above-mentioned preservative container, characterized by including: a first bag for storing blood from a donor; a first tube for transmitting the blood from the first bag; a filter for filtering out a predetermined blood cell component from the blood transmitted via the first tube; a second tube for transmitting the post-filtration blood that has been filtered through the filter; a second bag for storing the post-filtration blood transmitted via the second tube; a third tube for transmitting part of a blood component after centrifugation of the post-filtration blood in the second bag; a third bag for storing the part of the blood component transmitted via the third tube; the preservative container for containing the red blood cell preservative to be added to the residual blood component after centrifugation of the post-filtration blood in the second bag; and a fourth tube for transmitting the red blood cell preservative in the preservative container to the second bag.

Because the blood bag system of the invention includes the preservative container, dextrose in the first aqueous solution will not be denatured by the heat from autoclave sterilization. Furthermore, adsorption of the preservative ingredients in the first aqueous solution is reduced, and thus the solubility of the organic ingredients such as adenine in the first aqueous solution will not decrease. Furthermore, the mixing of the first aqueous solution and the second aqueous solution contained in the preservative container happening after autoclave sterilization makes the red blood cell preservative have improved storability of red blood cells.

### Advantageous Effects of Invention

According to a red blood cell preservative of the invention, browning thereof by the heat from sterilization is sufficiently reduced, and the effectiveness thereof is not decreased and preparation thereof is easily done. Furthermore, according to a preservative container, a manufacturing method for red blood cell preservative, and a blood bag system of the invention, browning of the red blood cell preservative by the heat from sterilization is sufficiently reduced, and the effectiveness of the red blood cell preservative is not decreased and preparation thereof is easily done.

### Brief Description of Drawings

Fig. 1 is a schematic view showing the configuration of an embodiment of a preservative container of the invention.
Fig. 2 is a schematic view showing the configuration of an embodiment of a preservative container of the invention.
Fig. 3 is a schematic view showing the configuration of an embodiment of a blood bag system of the invention. Description of Embodiments

Embodiments of a red blood cell preservative of the invention will now be described in detail.

A red blood cell preservative of the invention includes a first aqueous solution containing dextrose and a second aqueous solution containing disodium phosphate, which first and second aqueous solutions are mixed together after autoclave sterilization.

Here, "after autoclave sterilization" means immediately after the sterilization of a preservative container (to be described later), but also includes when the two solutions are added to concentrated red blood cells prepared in a blood bag system (to be described later). That is, the red blood cell preservative after autoclave sterilization may be in a state in which the first aqueous solution and the second aqueous solution are either mixed together or separated.

### (First aqueous solution)

The first aqueous solution is an aqueous solution containing mannitol, adenine, sodium citrate, sodium dihydrogen phosphate, and dextrose. The volume of the first aqueous solution is from 50 to 90 mL. As mentioned earlier, the first aqueous solution is constituted of the preservative ingredients that keep its pH within an acidic range rather than an alkaline range, such that denaturation of dextrose by the heat from autoclave sterilization can be reduced.

The pH of the first aqueous solution is preferably from 4.5 to 6.5. In order for the first aqueous solution to be prepared having the pH within the preceding range, the concentrations of the preservative ingredients are preferably in the following ranges:
mannitol: 5.0 to 10.0 g/L
adenine: 0.1 to 0.2 g/L
sodium citrate: 5.0 to 7.0 g/L
sodium dihydrogen phosphate: 0.01 to 0.10 g/L, and
dextrose: 5.0 to 7.0 g/L.

### (Second aqueous solution)

The second aqueous solution is an aqueous solution containing disodium phosphate. The volume of the second aqueous solution is from 10 to 50 mL. In order for the pH of the red blood cell preservative as a resultant mixture of the first aqueous solution and the second aqueous solution to be biologically acceptable 5.0 to 7.0, when the pH of the first aqueous solution is from 4.5 to 6.5, that of the second aqueous solution is preferably from 7.0 to 9.0. In order for the second aqueous solution to be prepared having the pH within the preceding range, the concentration of disodium phosphate is preferably from 2.0 to 4.0 g/L.

Now, a first embodiment of a preservative container of the invention will be described in detail.

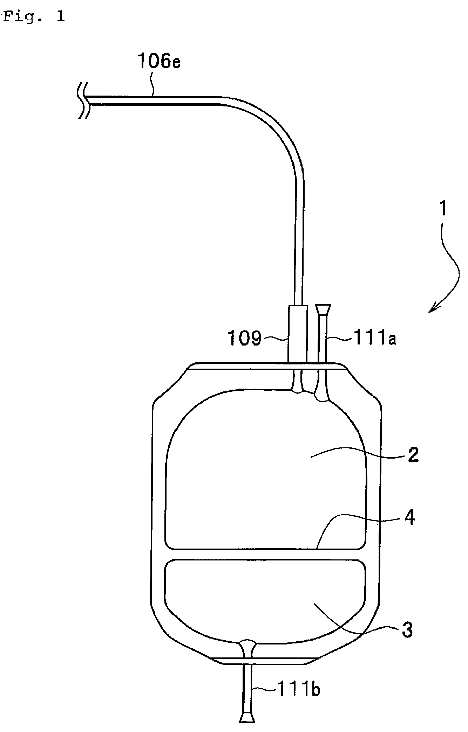

As shown in Fig. 1, a preservative container 1 of the invention is a bag-type container for containing the red blood cell preservative, of which container is formed of a flexible material; for example, a resin sheet is superposed on another resin sheet, both formed of polyvinyl chloride or the like, and the periphery thereof is heat sealed (sealed) to form a bag. Furthermore, the preservative container 1 includes a peelable weak seal 4 for partitioning the internal space of the bag-type container into a first preservative chamber 2 and a second preservative chamber 3.

### (First preservative chamber)

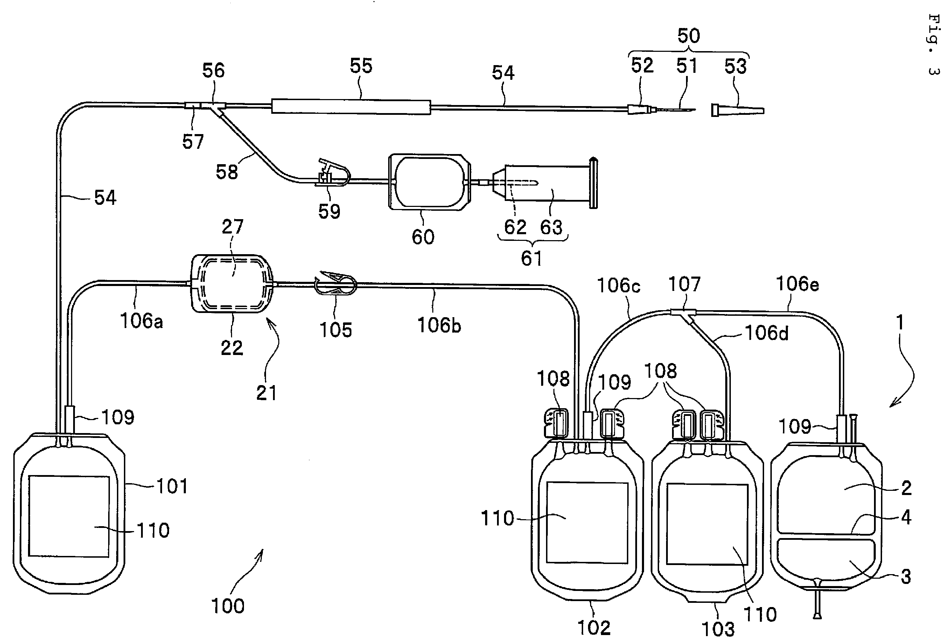

The first preservative chamber 2 contains a first aqueous solution (red blood cell preservative), and is from 50 to 90 mL in volume. The first preservative chamber 2 has an infusion tube 111a in the periphery, through which the first aqueous solution is infused into the internal space thereof. After completion of the infusion of the first aqueous solution, the end of the infusion tube 111a is sealed by heat sealing so as to prevent leakage therefrom. Furthermore, when the preservative container 1 is used in a blood bag system 100 (see Fig. 3) that will be described below, the first preservative chamber 2 is connected to a fourth tube 106e via a breakable seal member 109, which will be described below, to enable connection with another bag.

### (Second preservative chamber)

The second preservative chamber 3 contains a second aqueous solution (red blood cell preservative), and is from 10 to 90 mL in volume. The second preservative chamber 3 has an infusion tube 111b in the periphery, through which the second aqueous solution is infused into the internal space thereof. After completion of the infusion of the second aqueous solution, the end of the infusion tube 111b is sealed by heat sealing so as to prevent leakage therefrom. Furthermore, although not shown, when the preservative container 1 is used in the blood bag system 100, the second preservative chamber 3 instead of the first preservative chamber 2 may be connected to the fourth tube 106e via the breakable seal member 109 to enable connection with another bag.

### (Weak seal)

The weak seal 4 partitions the internal space of the preservative container 1 into the first preservative chamber 2 and the second preservative chamber 3 of predetermined volumes, respectively. The direction of partition in the internal space relative to the preservative container 1 that is in a suspended state, is not limited to be vertical; however, it may also be lateral (not shown). Furthermore, when the partitioning direction is lateral, the position of the first preservative chamber 2 in the preservative container 1 is not limited to be the upper but it may also be the lower. Likewise, when the partitioning direction is vertical, the position of the first preservative chamber 2 in the preservative container 1 may be either the left or the right, as well.

The weak seal 4 is formed by sealing the container walls together, specifically, by sealing the resin sheets of the preservative container 1 together, using heat sealing. It should be noted that, the forming of the weak seal 4 is performed prior to the infusion of the first aqueous solution and the second aqueous solution. Furthermore, the weak seal 4 is peelable as the first preservative chamber 2 or the second preservative chamber 3 is depressed. When the first preservative chamber 2 or the second preservative chamber 3 is depressed, the first aqueous solution or the second aqueous solution is pressurized, thereby peeling the weak seal 4.

The seal strength of the weak seal 4 is set so that the weak seal 4 peels off as the first preservative chamber 2 or the second preservative chamber 3 is depressed; and is preferably 1 - 25 N per 10 mm of width. If the seal strength is less than 1 N/10 mm, the weak seal 4 can already peel off before autoclave sterilization, causing mixing of the first aqueous solution and the second aqueous solution; consequently, the mixture is more subject to browning by the heat from sterilization. If the seal strength exceeds 25 N/10 mm in width, the peeling of the weak seal 4 will require a larger force, in particular, the preservative chamber must be depressed with a larger force, which can increase the workload of an operator.

Thus, in the preservative container 1, by the peeling of the weak seal 4 after autoclave sterilization, the first aqueous solution contained in the first preservative chamber 2 and the second aqueous solution contained in the second preservative chamber 3 are mixed together to make a red blood cell preservative.

Next, a second embodiment of the preservative container of the invention will be described in detail.

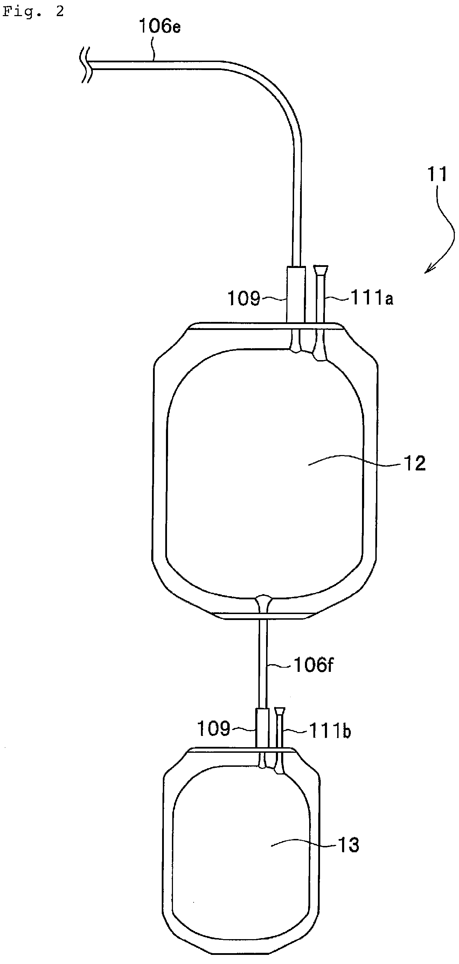

As shown in Fig. 2, the preservative container 11 of the invention contains the above-mentioned red blood cell preservative, and includes a first bag-type container 12, a second bag-type container 13, and a connecting tube 106f.

### (First bag-type container)

The first bag-type container 12 is a bag-type container for containing the above-mentioned first aqueous solution, in which bag-type container is formed of a flexible material; for example, a resin sheet is superposed on another resin sheet, both formed of polyvinyl chloride or the like, and the periphery thereof is heat sealed to form a bag. The volume of the first bag-type container 12 is from 50 to 90 mL. Further, the first bag-type container 12 has an infusion tube 111a in the periphery, through which the first aqueous solution is infused into the internal space thereof. After completion of the infusion of the first aqueous solution, the end of the infusion tube 111a is sealed by heat sealing so as to prevent leakage therefrom. Furthermore, although not shown, when the preservative container 11 is used in a blood bag system 100 that will be described below, the first bag-type container 12 is connected to a fourth tube 106e via a breakable seal member 109 to enable connection with another bag.

### (Second bag-type container)

The second bag-type container 13 is a bag-type container for containing the above-mentioned second aqueous solution, which bag-type container is formed of a flexible material; for example, a resin sheet is superposed on another resin sheet, both formed of polyvinyl chloride or the like, and the periphery thereof is heat sealed to form a bag. The volume of the second bag-type container 13 is from 10 to 50 mL. Further, the second bag-type container 13 has an infusion tube 111b in the periphery, through which the second aqueous solution is infused into the internal space thereof. After completion of the infusion of the second aqueous solution, the end of the infusion tube 111b is sealed by heat sealing so as to prevent leakage therefrom. Furthermore, although not shown, when the preservative container 11 is used in the blood bag system 100, the second bag-type container 13 instead of the first bag-type container 12 may be connected to the fourth tube 106e via the breakable seal member 109 to enable connection with another bag.

### (Connecting tube)

The connecting tube 106f, which interconnects the first bag-type container 12 and the second bag-type container 13, is a conventionally known tube that is used to transmit blood, which is formed of a flexible material, for example, polyvinyl chloride or the like. The connecting tube 106f is connected to the lower end of the first bag-type container 12 and to the upper end of the second bag-type container 13. The connecting position of the connecting tube 106f is not limited to that shown in Fig. 2; for example, the tube 106f may be connected to the upper end of the first bag-type container 12 and to the lower end of the second bag-type container 13 (not shown).

Preferably, the connecting tube 106f is provided with a breakable seal member 109 for opening and closing its passage. This will make it easy to prevent mixing of the first aqueous solution contained in the first bag-type container 12 and the second aqueous solution contained in the second bag-type container 13 prior to autoclave sterilization. As a result, browning of the red blood cell preservative is preventable with ease. The breakable seal member 109 may be disposed at other location along the connecting tube 106f than in the middle, for example, at the end of the first bag-type container 12 side or at the end of the second bag-type container 13 side. The breakable seal member 109 has a cover tube and a cylindrical body contained within the cover tube, having a solid tip for occluding the passage; and is configured to occlude the passage within the cover tube before breakage of the solid tip of the cylindrical body and to open the passage within the cover tube upon the breakage.

Thus, in the preservative container 11, after autoclave sterilization, the first aqueous solution contained in the first bag-type container 12 and the second aqueous solution contained in the second bag-type container 13 are mixed together via the connecting tube 106f upon passage opening by breakage of, if disposed, the breakable seal member 109, such that a red blood cell preservative is made.

Next, a manufacturing method for red blood cell preservative of the invention will be described in detail.

The manufacturing method for red blood cell preservative of the invention includes the following three steps when a preservative container 1 as shown in Fig. 1 is used for manufacturing red blood cell preservative.

### (First step)

In a first step, a first aqueous solution is contained in a first preservative chamber 2 of the preservative container 1 and a second aqueous solution is contained in a second preservative chamber 3 of the preservative container 1.

Here, the containing of the first aqueous solution and the second aqueous solution may be performed in no particular order, that is, the containing of the two solutions may be performed one after the other or simultaneously. The method for containing the solutions is one of conventionally known methods, where each solution is infused into the respective preservative chamber from a dispenser or the like via an infusion tube. Specifically, the first aqueous solution is infused into the first preservative chamber 2 via an infusion tube 111a, while the second aqueous solution is infused into the second preservative chamber 3 via an infusion tube 111b.

### (Second step)

Following the first step is a second step, in which the preservative container 1 containing the first aqueous solution and the second aqueous solution is autoclaved. Here, the method for sterilization is one of conventionally known methods using an autoclave or the like, and the conditions for sterilization are, for example, 20 minutes at 120 degrees Celsius.

### (Third step)

Following the second step is a third step, in which the first preservative chamber 2 or the second preservative chamber 3 is depressed to peel a seal 4 such that the first aqueous solution and the second aqueous solution are mixed together. Here, the method for depressing is one of conventionally known methods, for example, as the preservative container 1 is placed on a table or the like or it is suspended from a stand or the like, the first preservative chamber 2 or the second preservative chamber 3 is depressed with hand or the like such that the pressure of the first aqueous solution or the second aqueous solution is loaded to the seal 4. It should be noted that a conventionally known depressing apparatus may be used for the depressing. Thus, the first aqueous solution and the second aqueous solution are mixed together to make a red blood cell preservative that will improve the storability of red blood cells.

A manufacturing method for red blood cell preservative of the invention also includes the following three steps when a preservative container 11 as shown in Fig. 2 is used for manufacturing red blood cell preservative.

### (First step)

In a first step, a first aqueous solution is contained in a first bag-type container 12 and a second aqueous solution is contained in a second bag-type container 13 of the preservative container 11. Here, the order for containing the solutions and the containing method are the same as for the above-mentioned preservative container 1.

### (Second step)

Following the first step is a second step, in which the preservative container 11 containing the first aqueous solution and the second aqueous solution is autoclaved. Here, the sterilization method is the same as for the above-mentioned preservative container 1.

### (Third step)

Following the second step is a third step, in which the first aqueous solution and the second aqueous solution are mixed together via a connecting tube 106f interconnecting the first bag-type container 12 and the second bag-type container 13. Here, the mixing of the first aqueous solution and the second aqueous solution may take place either in the first bag-type container 12 or in the second bag-type container 13. Further, when a breakable seal member 109 is disposed at an end of the connecting tube 106f, the breakable seal member 109 is first broken to open the passage therewithin, and then the first aqueous solution and the second aqueous solution are mixed together. Thus, the first aqueous solution and the second aqueous solution are mixed together to make a red blood cell preservative that will improve the storability of red blood cells.

When the preservative container 11 is used to manufacture the red blood cell preservative, the following fourth step may be included in addition to the above three steps.

### (Fourth step)

Following the third step is a fourth step, in which the connecting tube 106f is cut to separate the second bag-type container 13 from the first bag-type container 12. Here, the cutting of the connecting tube 106f is performed using heat sealing or the like. Then, the cut end of the connecting tube 106f after the cutting is sealed using heat sealing or the like. Further, the bag-type container of the first bag-type container 12 and the second bag-type container 13 which was not used for solution mixing is disposed of.

Next, an embodiment of a blood bag system of the invention will be described in detail.

As shown in Fig. 3, the blood bag system 100 includes a first bag 101, a first tube 106a, a filter 21, a second tube 106b, a second bag 102, third tubes 106c, 106d, a third bag 103, a fourth tube 106e, and a preservative container 1. Alternatively, the blood bag system 100 may use a preservative container 11 instead of the preservative container 1. It should be noted that the preservative containers 1, 11 will not be further described since they are as explained earlier.

### (First bag)

The first bag 101 is for storing blood from a donor, and is a bag-type container which has been made into a bag by superposing a resin sheet on another resin sheet, both formed of polyvinyl chloride or the like, and heat sealing the periphery thereof. The volume of the first bag 101 is from 200 to 1000 mL. Furthermore, the first bag 101 is preferably pre-filled with an anticoagulant solution(s) such as ACD, CPD, or the like.

A conventionally known blood collecting means is connected to the first bag 101. In the blood collecting means, a blood sampling tube 54 is connected to the first bag 101 to provide the donor blood thereinto, and the other end of the blood sampling tube 54 is connected to a puncture device 50 to be used to make a puncture into the donor and collect blood. The puncture device 50 includes a blood collection needle 51, a hub 52 to fix the blood collection needle 51, and a protector 53 to cover the needle tip of the blood collection needle 51. Furthermore, the blood collecting means may include a sample blood bag 60 in which the initial flow blood of the collected blood is drawn and stored, and a conventionally known needle injury protector 55 to prevent needle injury with the blood collection needle 51 after blood collection.

The sample blood bag 60 is of a kind conventionally known, and is connected to a diversion connector 56 disposed at some midpoint along the blood sampling tube 54 via a diversion tube 58. A clamp 59 for occluding the passage of the diversion tube 58 is provided on the diversion tube 58. Further, a breakable seal member 57 having the same configuration as the above-mentioned breakable seal member 109 is provided for the diversion connector 56 at the first bag 101 side, in order to prevent the initial flow blood from entering the first bag during collection of the initial flow blood. Further, the sample blood bag 60 is connected with a sampling port 61, through which the sample blood is drawn into a reduced-pressure blood sampling tube that is not shown. The sampling port 61 includes a needle assembly 62 and a holder 63 containing the reduced-pressure blood sampling tube to be connected to the needle assembly 62.

### (First tube)

The first tube 106a transmits blood from the first bag 101 to the filter 21, whose one end being connected to the first bag 101 and the other end being connected to the filter 21. The one end of the first tube 106a is connected to the first bag 101 via a breakable seal member 109. It should be noted that, for the first tube 106a, a conventionally known tube that is used to transmit blood, for example, a polyvinyl chloride tube preferably having the same material properties as the first bag 101, is employed.

### (Filter)

The filter 21 filters the blood transmitted from the first bag 101 via the first tube 106a to remove a predetermined blood cell component(s) from the blood, for example, white blood cells, or white blood cells and platelets. The filter 21 includes a housing 22 and a filter medium 27 for filtering the blood.

The housing 22 has a substantially circular (including elliptical) shape or a substantially rectangular shape in the cross section along the blood flow direction, and is formed of polycarbonate, polyvinyl chloride, or the like. The filter medium 27 is formed by a porous membrane formed of polyurethane or the like, or an unwoven fabric formed of polyethylene terephthalate or the like. Further, the filter medium 27 preferably has a predetermined surface zeta potential in order to improve the removability of the predetermined blood cell component(s).

### (Second tube)

The second tube 106b transmits the post-filtration blood exiting the filter 21 into the second bag 102, whose one end being connected to the filter 21 and the other end being connected to the second bag 102. Further, a clamp 105 for occluding the passage of the second tube 106b is provided on the second tube 106b. It should be noted that, for the second tube 106b, a conventionally known tube that is used to transmit blood, for example, a polyvinyl chloride tube preferably having the same material properties as the second bag 102, is employed.

### (Second bag)

The second bag 102 is for storing the post-filtration blood transmitted via the second tube 106b, and is a bag-type container having the same shape as the first bag 101 which has been made into a bag by superposing a resin sheet on another resin sheet, both formed of polyvinyl chloride or the like, and heat sealing the periphery thereof. It should be noted that the second bag 102 may have an outlet 108 for expelling the post-filtration blood stored therein.

### (Third tube)

After the post-filtration blood stored in the second bag 102 is centrifuged, the third tubes 106c, 106d transmit part of the separated blood component to the third bag 103. One end of the third tube 106c is connected to the second bag 102 and the other end thereof is connected to a branch tube 107. Preferably, the third tube 106c is connected to the second bag 102 via a breakable seal member 109. One end of the third tube 106d is connected to the branch tube 107 and the other end thereof is connected to the third bag 103. It should be noted that, for each of the third tubes 106c, 106d, and the branch tube 107, a conventionally known tube or tubing that is used to transmit blood, for example, a polyvinyl chloride tube or tubing preferably having the same material properties as the third bag 103, is employed. It should be noted that the breakable seal member 109 is as mentioned earlier.

### (Third bag)

The third bag 103 is for storing the part of the blood component transmitted via the third tubes 106c, 106d, and is a bag-type container having the same shape as the first bag 101 which has been made into a bag by superposing a resin sheet on another resin sheet, both formed of polyvinyl chloride or the like, and heat sealing the periphery thereof. It should be noted that the third bag 103 may have an outlet 108 for expelling the blood component stored therein.

### (Fourth tube)

The fourth tube 106e transmits the red blood cell preservative contained in the preservative container 1 to the second bag 102, such that the red blood cell preservative is added to the post-centrifugation residual blood component stored in the second bag 102. One end of the fourth tube 106e is connected to the branch tube 107 and the other end thereof is connected to the preservative container 1. In the transmitting of the red blood cell preservative to the second bag 102, the fourth tube 106e is used for the transmitting from the preservative container 1 to the branch tube 107, and the third tube 106c is used for the transmitting from the branch tube 107 to the second bag 102. Preferably, the fourth tube 106e is connected to the fourth bag 104 via a breakable seal member 109. For the fourth tube 106e, a conventionally known tube that is used to transmit blood, for example, a polyvinyl chloride tube preferably having the same material properties as the preservative container 1, is employed. The breakable seal member 109 is as mentioned earlier.

In the blood bag system 100, preferably, a label 110 indicating the content of a bag is attached on the surface of each of the first bag 101, the second bag 102, and the third bag 103.

Next, how to use the blood bag system will be explained with reference to an example of a blood processing method using a blood bag system 100 that includes a preservative container 1 (see Figs. 1 and 3).
(1) With a needle injury protector 55 being spaced away from a hub 52, a clamp 59 is kept opened such that a diversion tube 58 is maintained in an open state. Under this condition, blood collection is started by puncturing a blood collection needle 51 into a blood vessel of a donor. In this way, the initial flow blood will be drawn into a sample blood bag 60 via a blood sampling tube 54 and the diversion tube 58.
(2) After a predetermined amount of initial flow blood is collected in the sample blood bag 60, the clamp 59 is closed to occlude the diversion tube 58, and a breakable seal member 57 is broken to open a passage and turns the blood sampling tube 54 to an open state. Thus, the blood, from which the initial flow blood has been sequestered, is drawn into a first bag 101 via the blood sampling tube 54.
(3) After a predetermined amount of blood is collected in the first bag 101, blood collection is completed by withdrawing the blood collection needle 51 from the blood vessel of the donor.
(4) The needle injury protector 55 is moved toward the hub 52, or the blood sampling tube 54 is pulled toward the first bag 101 so as to retract the hub 52 (the blood collection needle 51), until the needle injury protector 55 comes to a state in which the puncture device 50 is contained and held within the needle injury protector 55.
(5) The blood sampling tube 54 and the diversion tube 58 are sealed by heat sealing using a tube sealer or the like. Then, the puncture device 50 and the sample blood bag 60 are cut away from the first bag 101, and the puncture device 50 is disposed of.
(6) A breakable seal member 109 for a first tube 106a is broken to open a passage, and the blood collected in the first bag 101 is transmitted to a filter 21 via the first tube 106a. In the filter 21, then, white blood cells and platelets are filtered out of the transmitted blood. A clamp 105 on a second tube 106b is opened, and the post-filtration blood is transmitted to and collected in a second bag 102 via a second tube 106b.
(7) The clamp 105 on the second tube 106b is closed, and the second tube 106b is sealed by heat sealing using a tube sealer or the like. The first bag 101 and the filter 21 are then cut away from the second bag 102.
(8) The second bag 102 is centrifuged such that the blood in the bag is separated into a layer of red blood cells and a layer of plasma. A breakable seal member 109 for a third tube 106c is broken to open a passage, and the plasma is transmitted to a third bag 103 via the third tubes 106c, 106d.
(9) A first preservative chamber 2 or a second preservative chamber 3 in the preservative container 1 is depressed to peel a weak seal 4. As a result, a first aqueous solution contained in the first preservative chamber 2 and a second aqueous solution contained in the second preservative chamber 3 are mixed together to make a red blood cell preservative. It should be noted that, the mixing of the first aqueous solution and the second aqueous solution may take place after autoclave sterilization in the course of manufacture of the preservative container 1. In this case, although not shown, the blood bag system 100 would include a preservative container 1 in which the weak seal 4 has been peeled off and the first preservative chamber 2 and the second preservative chamber 3 have been in communication with each other.
(10) The red blood cell preservative in the preservative container 1 is added to the residual concentrated red blood cells in the second bag 102 via the third tube 106c and a fourth tube 106e upon passage opening by breakage of, if disposed, a breakable seal member 109, and then mixed together. As a result, a concentrated red blood cell solution is prepared in the second bag 102, and platelet poor plasma is prepared in the third bag 103.
(11) In (6), if the filter 21 filters out white blood cells alone, platelet rich plasma would be prepared in the third bag 103 in (8). Hence, the third bag 103 may be further centrifuged to prepare platelet concentrated plasma in the third bag 103 and platelet poor plasma in the preservative container 1 which has been empty after the red blood cell preservative was transmitted out.

The same blood processing methods (1) to (11) applies also for a case in which a blood bag system that includes a preservative container 11 (see Fig. 2) is used.

In this case, in (9), the first aqueous solution contained in a first bag-type container 12 and the second aqueous solution contained in a second bag-type container 13 are mixed together via a connecting tube 106f upon passage opening by breakage of, if disposed, a breakable seal member 109, such that a red blood cell preservative is made. Further, the mixing of the first aqueous solution and the second aqueous solution is preferably taken place in one of the first bag-type container 12 and the second bag-type container 13, which is connected with the fourth tube 106e. In Fig. 2, the first aqueous solution and the second aqueous solution that has been transmitted via the connecting tube 106f are mixed in the first bag-type container 12. It should be noted that, the mixing of the first aqueous solution and the second aqueous solution may take place after autoclave sterilization in the course of manufacture of the preservative container 11. Further, after the mixing of the first aqueous solution and the second aqueous solution, the bag-type container that was not used for the mixing may be disposed of by cutting the connecting tube 106f. In this case, although not shown, the blood bag system 100 would include one of the first bag-type container 12 and the second bag-type container 13 as the preservative container 11.

In (10), the red blood cell preservative in the preservative container 11 (that is, the first bag-type container 12 or the second bag-type container 13) is added to the residual concentrated red blood cells in the second bag 102 via the third tube 106c and the fourth tube 106e upon passage opening by breakage of, if disposed, the breakable seal member 109, and then mixed together. Examples

Examples of the invention will now be described.

### (Examples)

Prepared were 90 mL of a test solution A and 10 mL of a test solution B, respectively containing preservative ingredients as shown in Table 1. Here, the test solution A corresponds to a first aqueous solution of a red blood cell preservative of the invention and the test solution B corresponds to a second aqueous solution of the red blood cell preservative of the invention.

### (Comparative example)

Prepared was 100 mL of a test solution C containing preservative ingredients as shown in Table 1. Here, the test solution C corresponds to a mixture of the first aqueous solution of the red blood cell preservative of the invention and the second aqueous solution of the red blood cell preservative of the invention, mixed prior to autoclave sterilization.

**[Table 1]**

| Preservative ingredients | Examples | | Comparative example |
|---|---|---|---|
| | Test solution A | Test solution B | Test solution C |
| | Ingredient concentration (g/L) | Ingredient concentration (g/L) | Ingredient concentration (g/L) |
| Mannitol | 6.016 | - | 6.016 |
| Adenine | 0.189 | - | 0.189 |
| Sodium dihydrogen phosphate | 0.078 | - | 0.078 |
| Disodium phosphate | - | 2.300 | 2.300 |
| Sodium citrate | 6.117 | - | 6.117 |
| Dextrose | 6.804 | - | 6.804 |

| | | | |
|---|---|---|---|
| Note: The symbol "-" indicates that the ingredient is not contained. | | | |

The test solutions A, B, and C shown in Table 1 were respectively filled in polyvinyl chloride bags, and then the bags were sterilized using an autoclave (available from Getinge). The sterilization condition was 20 minutes, 120 degrees Celsius. After sterilization, the color tones of the test solutions A, B, and C filled in the respective bags were visually examined. As a result, browning was not found in the Examples, which are the test solutions A and B. In contrast, browning was found in the comparative example, which is the test solution C.

### Reference Signs List

- 1: Preservative container
- 2: First preservative chamber
- 3: Second preservative chamber
- 4: Weak seal
- 11: Preservative container
- 12: First bag-type container
- 13: Second bag-type container
- 21: Filter
- 22: Housing
- 27: Filter medium
- 100: Blood bag system
- 101: First bag
- 102: Second bag
- 103: Third bag
- 104: Fourth bag
- 106a: First tube
- 106b: Second tube
- 106c: Third tube
- 106d: Third tube
- 106e: Fourth tube
- 106f: Connecting tube

## Claims

1. A red blood cell preservative, comprising:
a first aqueous solution containing mannitol, adenine, sodium citrate, sodium dihydrogen phosphate, and dextrose; and
a second aqueous solution containing disodium phosphate, wherein
the first aqueous solution and the second aqueous solution are mixed together after autoclave sterilization.

2. A preservative container for containing the red blood cell preservative according to claim 1, comprising:
a bag-type container formed of a flexible material, wherein
the bag-type container includes a peelable weak seal for partitioning the internal space of the bag-type container into a first preservative chamber and a second preservative chamber, wherein
the first preservative chamber contains the first aqueous solution and the second preservative chamber contains the second aqueous solution; and wherein
after autoclave sterilization, the first preservative chamber or the second preservative chamber is depressed to peel the weak seal such that the first aqueous solution and the second aqueous solution are mixed together.

3. A preservative container for containing the red blood cell preservative according to claim 1, comprising:
a first bag-type container that is formed of a flexible material and contains the first aqueous solution;
a second bag-type container that is formed of a flexible material and contains the second aqueous solution; and
a connecting tube that is formed of a flexible material and interconnects the first bag-type container and the second bag-type container, wherein
after autoclave sterilization, the first aqueous solution and the second aqueous solution are mixed together via the connecting tube.

4. A manufacturing method for red blood cell preservative, for manufacturing the red blood cell preservative using the preservative container according to claim 2, comprising:
a first step of containing the first aqueous solution in the first preservative chamber and the second aqueous solution in the second preservative chamber, respectively;
following the first step, a second step of autoclaving the preservative container containing the first aqueous solution and the second aqueous solution; and
following the second step, a third step of mixing the first aqueous solution and the second aqueous solution by peeling the seal by depressing the first preservative chamber or the second preservative chamber.

5. A manufacturing method for red blood cell preservative, for manufacturing the red blood cell preservative using the preservative container according to claim 3, comprising:
a first step of containing the first aqueous solution in the first bag-type container and the second aqueous solution in the second bag-type container, respectively;
following the first step, a second step of autoclaving the preservative container containing the first aqueous solution and the second aqueous solution; and
following the second step, a third step of mixing first aqueous solution and the second aqueous solution via the connecting tube.

6. The manufacturing method for red blood cell preservative according to claim 5, further comprising:
following the third step, a fourth step of separating the second bag-type container from the first bag-type container by cutting the connecting tube.

7. A blood bag system comprising the preservative container according to claim 2 or 3, comprising:
a first bag for storing blood from a donor;
a first tube for transmitting the blood from the first bag;
a filter for filtering out a predetermined blood cell component from the blood transmitted via the first tube;
a second tube for transmitting the post-filtration blood that has been filtered through the filter;
a second bag for storing the post-filtration blood transmitted via the second tube;
a third tube for transmitting part of a blood component after centrifugation of the post-filtration blood in the second bag;
a third bag for storing the part of the blood component transmitted via the third tube;
the preservative container for containing the red blood cell preservative to be added to the residual blood component after centrifugation of the post-filtration blood in the second bag; and
a fourth tube for transmitting the red blood cell preservative in the preservative container to the second bag.
